# EUROPÄISCHE PATENTSCHRIFT

(11) **EP 1 525 161 B1**
(45) Veröffentlichungstag und Bekanntmachung des Hinweises auf die Patenterteilung: **20.08.2008**
(21) Anmeldenummer: 03763882.2
(22) Anmeldetag: 17.07.2003
(51) Int. Cl.: C01B 39/02, B01J 29/06, C07D 301/12, C07B 41/02, C07C 249/08, C01B 21/14

(54) **VERFAHREN ZUR HERSTELLUNG EINES ZEOLITHHALTIGEN FESTSTOFFES**
METHOD FOR PRODUCING A SOLID CONTAINING ZEOLITES
PROCEDE POUR PRODUIRE UNE MATIERE SOLIDE CONTENANT DES ZEOLITHES

(30) Priorität: 17.07.2002 DE 10232406
(43) Veröffentlichungstag der Anmeldung: 27.04.2005
(73) Patentinhaber: BASF SE, 67056 Ludwigshafen (DE)
(72) Erfinder: MÜLLER, Ulrich, 67435 Neustadt (DE); VOSS, Hartwig, 67227 Frankenthal (DE); SCHUBERT, Erich, 67098 Bad Dürkheim (DE); HILL, Friedrich, 67149 Meckenheim (DE); PETERSEN, Hermann, 67269 Grünstadt (DE)
(74) Vertreter: Isenbruck, Günter
(86) Internationale Anmeldenummer: PCT/EP2003/007767
(87) Internationale Veröffentlichungsnummer: WO 2004/007369

(56) Entgegenhaltungen:
- EP-B- 0 607 276
- WO-A-98/55229
- US-A- 5 863 516

## Beschreibung

Die vorliegende Erfindung betrifft ein Verfahren zum Aufkonzentrieren eines mindestens einen Zeolithen enthaltenden, wenigstens teilweise kristallinen Feststoffes in einem Gemisch, wobei dieses Gemisch mindestens einen Hilfsstoff, beispielsweise eine Templat-Verbindung sowie den besagten Feststoff umfasst. Das Verfahren zeichnet sich insbesondere dadurch aus, dass das Gemisch in einem Schritt (II) durch Ultrafiltration in ein Retentat und ein Permeat aufgeteilt wird, wobei der Gehalt an Feststoff im Retentat höher ist als im Gemisch und der Gehalt an Feststoff im Permeat geringer ist als im Gemisch. Diese Verfahrensführung erlaubt das Rückführen von im Permeat befindlichen Hilfsstoffen, insbesondere von Templat-Verbindungen, in einen dem Schritt (II) vorgelagerten Kristallisations-Schritt (I).

Integrierte Verfahren zur Herstellung zeolithhaltiger Formkörper sind beispielsweise in Druckschriften der Anmelderin beschrieben, so insbesondere in der WO 98/55229. Der Schwerpunkt dieser Druckschrift liegt auf der Auswahl des Bindemittels, mit Hilfe dessen der zeolithhaltige Feststoff zu einem Formkörper kompaktiert werden kann. Die WO 98/55229 offenbart keine Verfahren zum Aufkonzentrieren des in der Mutterlauge befindlichen Feststoffes, welche nicht durch die konventionellen Verfahren der Filtration und/oder des Zentrifugierens abgedeckt wären.

Die US 6 106 803 beschreibt ein Verfahren zur Herstellung von Titansilikalit-Granulaten, in welchem ein Synthesegel (= Synthese-Gemisch; Si- und Ti-Quelle, Templatbildner, Base und Wasser) unter hydrothermalen Bedingungen kristallisiert wird, und die dabei entstehende zeolithische Suspension gegebenenfalls nach Aufkonzentrieren und/oder dem Zusatz weiterer Stoffe einer Sprühtrocknung oder einer Wirbelschicht-Sprühgranulationstrocknung unterzogen wird. Dabei liegt der Feststoffgehalt im Gemisch vor dem Schritt des Sprühtrocknens in einem Bereich um 10%. Ein solcher, vergleichsweise niedriger Feststoff-Gehalt führt letztlich im Formkörper zu einer unnötig erniedrigten massenspezifischen katalytischen Aktivität, insbesondere, wenn dem Gemisch zum Granulieren Zusatzstoffe zugegeben werden.

Die EP-B 0 638 362 betrifft u.a. ein Verfahren zum Herstellen eines Titansilicalit-Katalysators, und dabei insbesondere das Agglomerieren der Primärpartikel, d.h. der Mikro-Partikel, die sich im Kristallisations-Schritt der Titansilicalit-Synthese bilden. Dieses Agglomerieren wird erreicht durch Erniedrigen des pH-Wertes der Lösung, enthaltend die Primärpartikel (zeolithische Suspension), und zwar auf Werte von pH 5 bis zu pH 10. Das Agglomerieren ist Teil eines integrierten Verfahrens, in welchem (i) zunächst die Primärpartikel aus einem Synthese-Gemisch nach dem Stand der Technik hergestellt werden, anschließend (ii) die Sekundärpartikel wie oben erwähnt durch Erniedrigen des pH-Wertes agglomeriert werden sowie (iii) die Sekundärpartikel abschließend kalziniert werden. Die EP-B 0 638 362 erteilt allerdings keine über den Stand der Technik hinausreichende Lehre bezüglich des Aufkonzentrierens von Primär- oder Sekundärpartikeln vor dem Agglomerieren und/oder des Rückgewinnens von Bestandteilen der Mutterlauge.

Auch die US 4 701 428 befasst sich mit der Problematik des Agglomerierens in einem Gemisch, enthaltend zeolithische Mikrokristalle (hier: kleiner als 5 µm). Das Problem wird durch eine spezielle Verfahrensführung zum Agglomerieren von Titansilicalit gelöst. Dieses umfasst die Zugabe von Titansilicalit-Kristallen zu einer wässrigen Lösung, enthaltend Tetraalkyl-Orthosilikate, bei definierten Temperaturen sowie ein schnelles Trocknen. Auch diese Druckschrift erteilt keine über den Stand der Technik hinausreichende Lehre bezüglich des Aufkonzentrierens von Titansilicalit-Kristallen und/oder des Rückgewinnens von Bestandteilen der Mutterlauge. Ein ähnliches Verfahren ist auch in der EP-B 1 106 576 beschrieben.

Die EP 0 607 276 B 1 betrifft ein Verfahren zur Behandlung von Zeolithkristallen, bei dem die Mutterlauge, die die Zeolithkristalle enthält, durch Mikrofiltration aufkonzentriert wird. Hierbei wird eine wässrige Aufschlämmung der Zeolithkristalle durch einen Mikofilter zirkulieren gelassen, während Permeat entfernt wird, um die Aufschlämmung vor einem folgenden Waschschritt zu konzentrieren. Nach dem Waschen wird die wässrige Aufschlämmung durch erneutes Entfernen von Permeat weiter aufkonzentriert. Hierbei enthält EP 0 607 276 B 1 keine Lehre über die Rückgewinnung von Bestandteilen der Mutterlauge durch Rückführen des Permeats.

Aufgabe der vorliegenden Erfindung war es, ein Verfahren zum Aufkonzentrieren des aus der mindestens teilweisen Kristallisation eines Synthese-Gemisches resultierenden Gemisches, enthaltend wenigstens einen Hilfsstoff, beispielsweise eine Templat-Verbindung in einer Mutterlauge, sowie einen zumindest teilweise kristallinen Anteil an Feststoffen, enthaltend wenigstens ein zeolithisches Material, bereitzustellen. In diesem Verfahren soll der Feststoff-Gehalt im Gemisch erhöht werden und gleichzeitig die templathaltige Mutterlauge zumindest teilweise vom Feststoff abgetrennt werden. Ein erhöhter Feststoff-Gehalt führt letztlich zu einer höheren massenspezifischen Katalysatoraktivität.

Das integrierte Verfahren gemäß Anspruch 1 soll es ermöglichen, die Herstellung zeolithhaltiger Formkörper insgesamt zu vereinfachen, z. B. durch den Wegfall von Zwischen-Kalzinier-Schritten. Weiterhin soll das Verfahren dazu führen, den Verbrauch an potenziell kostenintensiven oder umweltschädigenden Chemikalien, wie beispielsweise von Templatbildnern, zu minimieren.

Diese Aufgabe wird dadurch gelöst, dass nach einem Schritt (I) ein Gemisch (I), enthaltend zumindest einen Hilfsstoff sowie den zumindest teilweise kristallinen zeolithhaltigen Feststoff, in einem Schritt (II) durch Ultrafiltration in ein Retentat und ein Permeat aufgeteilt wird, d.h. aufkonzentriert wird, wobei der Gehalt an Feststoff im Retentat höher ist als im aus Schritt (I) entstammenden Gemisch (I) und der Gehalt an Feststoff im Permeat geringer ist als in besagtem Gemisch. Diese Verfahrensführung erlaubt das Rückführen von im Permeat befindlichen Hilfsstoffen, insbesondere von Templat-Verbindungen in den oben erwähnten Kristallisations-Schritt (I).

Der in der vorliegenden Erfindung beschriebene Feststoff kann in einem weiteren Schritt zu einem Formkörper kompaktiert werden, der insbesondere als Katalysator zur Epoxidation von organischen Verbindungen eingesetzt werden kann.

Das erfindungsgemäße integrierte Verfahren kann auch Teil eines Verfahrens zur Herstellung eines abriebfesten Formkörpers, der mindestens ein zeolithisches Material enthält, sein. Ein solches Verfahren lässt sich rein schematisch in die folgenden Schritte unterteilen:
- Schritt (I):: zumindest teilweise Kristallisation eines mindestens einen Zeolithen enthaltenden Feststoffes aus einem Synthese-Gemisch unter Erhalt des Gemisches (I), umfassend zumindest den besagten Feststoff sowie mindestens einen Hilfsstoff;
- Schritt (II):: Aufkonzentrieren des Feststoffes, der sich im Gemisch (I) befindet, durch Ultrafiltration unter Erhalt eines Retentats und eines Permeats; dieser Schritt beinhaltet optional eine Fest-Flüssig-Trennung, beispielsweise des Feststoffes von der Mutterlauge;
- Schritt (III):: Agglomerieren oder Granulation oder Agglomerieren und Granulation der Feststoff-Partikel im aufkonzentrierten Retentat aus Schritt (II); dieser Schritt beinhaltet optional das Trocknen der Feststoff-Partikel
- Schritt (F):: Formgebung, anschließend an die Schritte (II) oder (III);
- Schritt (K):: Kalzinierung, anschließend an die Schritte (III) oder (F),
wobei die Schritte (F) und (K) in jedem Fall optional sind.

In der vorliegenden Anmeldung wird der erfindungsgemäß hergestellte zeolithhaltige Feststoff bzw. der daraus erhältliche Formkörper im Zusammenhang mit Anwendungen in der Katalyse diskutiert. Dies kann aber in keiner Weise dahingehend gedeutet werden, dass der Feststoff und/oder der Formkörper nicht auch in anderen Anwendungen oder Zusammenhängen eingesetzt werden kann.

Demgemäß betrifft die vorliegende Erfindung ein integriertes Verfahren zur Herstellung eines mindestens einen Zeolithen enthaltenden Feststoffes, umfassend mindestens die folgenden Schritte
- Schritt (I): zumindest teilweise Kristallisation eines mindestens einen Zeolithen enthaltenden Festsstoffes aus einem Synthese-Gemisch unter Erhalt des Gemisches (I), umfassend zumindest den besagten Feststoff sowie mindestens einen Hilfsstoff;
- Schritt (II): Aufkonzentrieren des Feststoffes, der sich im Gemisch (I) befindet, durch Ultrafiltration unter Erhalt eines Retentats und eines Permeats;
- Schritt (III): Agglomerieren oder Granulation oder Agglomerieren und Granulation der Feststoff-Partikel im Retentat aus Schritt (II),
dadurch gekennzeichnet, dass das im Schritt (II) erhaltene Permeat oder zumindest ein darin enthaltener Hilfsstoff zumindest teilweise in den Schritt (I) zurückgeführt wird.

Im Folgenden sollen die wesentlichen Begriffe, wie sie im Rahmen der vorliegenden Erfindung verwendet werden, definiert werden.

Unter einem "Synthese-Gemisch" im Sinne der vorliegenden Erfindung ist jedes Gemisch zu verstehen, aus welchem durch Kristallisation ein in einem Gemisch, vorzugsweise einer Mutterlauge, suspendierter Feststoff erhalten werden kann, wobei der Feststoff (i) zumindest teilweise kristallin sein sollte und (ii) zumindest ein zeolithisches Material enthalten sollte. Das Synthese-Gemisch kann als Sol, als Gel, als Lösung oder als Suspension vorliegen.

"Zeolithe" sind kristalline Alumosilicate mit geordneten Kanal- und Käfigstrukturen, die Mikroporen aufweisen. Die Begriffe "Mikroporen", wie er im Rahmen der vorliegenden Erfindung verwendet wird, entspricht der Definition in "Pure Appl. Chem." 45, S. 71 ff., insbesondere S. 79 (1976), und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise in W.M. Meier und D.H. Olson in "Atlas of Zeolite Structure Types", Elsevier, 4. Auflage, London 1996. Neben Mikroporen können die erfindungsgemäßen Feststoffe, die mindestens einen Zeolithen enthalten, auch Mesoporen und/oder Makroporen aufweisen.

Unter einem "Feststoff", wie er beispielsweise nach der Kristallisation aus dem Synthese-Gemisch vorliegt, ist im Sinne der vorliegenden Erfindung jedes nicht-molekulare Material zu verstehen, welches (i) mindestens ein zeolithisches Material enthält sowie (ii) solchermaßen als Phase vom Gemisch (I) verschieden ist, dass es einem Verfahren der Abtrennung und/oder einem Verfahren des Aufkonzentrierens zugänglich ist. Der Feststoff liegt dabei typischerweise als Partikel suspendiert in einer Mutterlauge vor, wobei die Partikelgröße durch die Größe der Teilchen gegeben ist, die durch den im erfindungsgemäßen Verfahren verwendeten Membranfilter (bei Ultra- bzw. Diafiltration) gerade noch erfasst werden. Dabei soll die Größe der noch als Feststoff anzusehenden Partikel mindestens 2 nm betragen. Ein Feststoff kann als "Primärpartikel" (nach der Kristallisation) oder als "Sekundärpartikel" (nach einem Schritt des Agglomerierens und/oder der Granulation) vorliegen.

Unter einer "Mutterlauge" im Sinne der vorliegenden Erfindung ist jede flüssige Phase zu verstehen, die beliebige Substanzen in gelöster Form enthalten kann, jedoch frei ist von Partikeln, die größer sind als 2 nm. Bis zu 5 Gew.-% an Partikeln größer als 2 nm können dabei in der Mutterlauge enthalten sein. Dabei kann die Mutterlauge im Sinne der vorliegenden Erfindung nicht abreagierte Bestandteile des Synthese-Gemisches, also Hilfsstoffe, enthalten, so beispielsweise mindestens eine Verbindung, die im Schritt (I) als Templatbildner zur Synthese der zeolithhaltigen Feststoffes eingesetzt wurde. Im Sinne der vorliegenden Erfindung liegt eine Mutterlauge erst nach Beendigung des Schrittes (I) vor, d.h. typischerweise im Zusammenhang mit einer Suspension, die Feststoff-Partikel der oben definierten Art enthält. Im Schritt (II) besteht das Permeat im wesentlichen aus Mutterlauge.

Ein "Templatbildner" im Sinne der vorliegenden Erfindung ist jede Substanz, die dazu führt, dass bei der Ausbildung des mindestens einen zeolithischen Materials aus dem Synthese-Gemisch der entstehende Feststoff über mindestens einen Typ von Poren (Mikro-, Meso- oder Makroporen) verfügt. Typischerweise werden stickstoffhaltige organischen Basen eingesetzt, wobei dies nicht als Beschränkung, sondern als illustrierendes Beispiel zu verstehen ist.

Der Schritt (II) der vorliegenden Erfindung betrifft das "Aufkonzentrieren" des feststoffhaltigen Gemisches aus Schritt (I). Dabei ist unter "Aufkonzentrieren" im Sinne der vorliegenden Erfindung jeder Schritt zu verstehen, der zum Resultat führt, dass am Ende des besagten Schrittes ein Gemisch steht, in welchem der Gehalt an Feststoff gegenüber dem ursprünglich eingesetzten Gemisch erhöht ist. Bei den Gemischen kann es sich, muss es sich aber nicht um Suspensionen des Feststoffes handeln. Ein "Abtrennen" des Feststoffes vom Gemisch bzw. von der Suspension ist explizit im Definitionsbereich von "Aufkonzentrieren" als Spezialfall enthalten.

Unter einem "Formkörper" im Sinne der vorliegenden Erfindung ist jeder dreidimensionale Körper zu verstehen, der in einem wie unten näher beschriebenen Formgebungsschritt (F) entstanden ist. Der Formkörper wird dabei typischerweise durch Kompaktieren eines Feststoffes erhalten. Dieser Feststoff wiederum ist erhältlich aus den Schritten (II) und/oder (III) unter optionaler Kalzinierung (K).

Bei der "Ultrafiltration", wie sie in der vorliegenden Erfindung eingesetzt wird, handelt es sich um einen konvektiven Prozess, bei welchem die Trennung von Teilchen (Partikel, Makromoleküle etc.) und Lösungsmittel primär aufgrund der Teilchengröße (und nur wenig beeinflusst durch die Ladung der Teilchen) erfolgt. Dabei wird ein Druckgradient entlang einer typischerweise anisotropen, semipermeablen Membran angelegt. Je kleiner die Porengröße der Membran, desto größer ist der zum Aufkonzentrieren notwendige Energieaufwand, gegeben durch den anzulegenden Druckgradienten. Verfahren der Mikrofiltration, d.h. Filtration unter Verwendung von Membranen mit Porendurchmesser im Mikrometer-Bereich sind explizit in das erfindungsgemäße Verfahren der Ultrafiltration mit eingeschlossen, solange die Abgrenzung zur konventionellen Kuchenfiltration wie unten definiert, gegeben ist.

Die "Diafiltration" unterscheidet sich nicht prinzipiell von der Ultrafiltration, insbesondere werden auch in diesem Fall die unten näher beschriebenen Membranen mit den dort spezifizierten Porengrößen verwendet. Diafiltration ist im Unterschied zur Ultrafiltration durch eine andere Verfahrensführung gekennzeichnet, nämlich dadurch, dass das Permeat (siehe unten angegebene Definition) kontinuierlich oder teilweise durch Wasser oder eine andere Lösung ersetzt wird. Dabei handelt es sich dann um einen Reinigungsschritt, der im Sinne der vorliegenden Erfindung optional nach einem Schritt des Aufkonzentrierens, d.h. der Ultrafiltration erfolgen kann.

Ultrafiltration im Sinne der vorliegenden Erfindung beinhaltet die Verwendung von Membranen mit Porengrößen von 1 nm bis 1 µm. Damit unterscheidet sich die hier beanspruchte Art der Filtration zum Zweckes des Aufkonzentrierens, d.h. des Erhöhens des Feststoff-Gehaltes, klar vom Stand der Technik, der durch Kuchenfiltration und Zentrifugieren des zeolithhaltigen Gemisches gegeben ist. Die/das durch diese Techniken zu erreichende Abtrennung/Aufkonzentrieren wirkt effektiv nur für Feststoff Partikel größer als 10 µm.

Die Ultra- bzw. Diafiltration trennt das ursprünglich eingesetzte Gemisch in zwei voneinander verschiedene, d.h. abtrennbare Phasen: das Permeat und das Retentat. Im Sinne der vorliegenden Erfindung ist unter einem "Permeat" der Teil des nach dem Schritt (II) verbleibenden Gemisches zu verstehen, der auf der Rückseite der Membran, d.h. auf der Seite des geringeren Druckes abgezogen wird (bei der konventionellen Filtration würde man von dem "Filtrat" sprechen). Entsprechend entsteht das "Retentat" auf der Seite der Membran, die dem höheren Druck ausgesetzt ist und in welcher sich die Feststoff-Partikel, die nicht durch die Poren der Membran passen, aufkonzentrieren.

Die im Rahmen der vorliegenden Erfindung verwendeten Begriffe "Granulation" und "Agglomerieren" sind als synonym zu betrachten und bezeichnen jedes denkbare Verfahren, nach welchem der Durchmesser eines Partikels vergrößert werden kann. Die Vergrößerung kann durch Zusammenbacken von Teilchen oder durch Aufwachsen weiterer Lagen geschehen. Das Granulieren umfasst dabei, ist aber nicht beschränkt auf, Verfahren, in welchen das Benetzen der Partikel durch mindestens eine Flüssigkeit eine Rolle spielt. Weiterhin können, müssen aber nicht, Bindemittel zugesetzt werden, die das Agglomerieren bzw. Granulieren fördern oder möglich machen.

Im Folgenden sind die einzelnen Schritte des integrierten Verfahrens zum Herstellen eines Formkörpers, enthaltend wenigstens ein zeolithisches Material, sowie die zugehörigen Ausführungsformen beschrieben. Von besonderer Bedeutung ist dabei das erfindungsgemäße Verfahren der vorliegenden Anmeldung, welches im wesentlichen Schritt (II) entspricht. Wie bereits oben erwähnt, lässt sich das integrierte Verfahren rein schematisch in die folgenden Teilschritte zerlegen:
- Schritt (I):: zumindest teilweise Kristallisation eines mindestens einen Zeolithen enthaltenden Festsstoffes aus einem Synthese-Gemisch unter Erhalt des Gemisches (I), umfassend zumindest den besagten Feststoff sowie mindestens einen Hilfsstoff;
- Schritt (II):: Aufkonzentrieren des Feststoffes, der sich im Gemisch (I) befindet, durch Ultrafiltration unter Erhalt eines Retentats und eines Permeats; dieser Schritt beinhaltet optional eine Fest-Flüssig-Trennung, beispielsweise des Feststoffes von der Mutterlauge;
- Schritt (III):: Agglomerieren oder Granulation oder Agglomerieren und Granulation der Feststoff-Partikel im aufkonzentrierten Retentat aus Schritt (II); dieser Schritt beinhaltet optional das Trocknen der Feststoff-Partikel
- Schritt (F):: Formgebung, anschließend an die Schritte (II) oder (III);
- Schritt (K):: Kalzinierung, anschließend an die Schritte (III) oder (F).

### Schritt (I): Synthese-Gemisch

Bezüglich des mindestens einen Zeolithen, der im erfindungsgemäßen Feststoff oder Formkörper vorliegen soll, existieren keinerlei Beschränkungen. Vorzugsweise wird ein Titan-, Zirkonium-, Chrom-, Niob-, Eisen-, Bor- oder Vanadium-haltiger Zeolith und insbesondere ein Titansilicalit eingesetzt.

Solche Titanzeolithe, insbesondere solche mit einer Kristallstruktur von MFI-Typ, sowie Möglichkeiten zu Ihrer Herstellung sind beispielsweise in der WO 98/55228, WO 98/03394, WO 98/03395, EP-A 0 311 983 oder EP-A 405 978 beschrieben. Außer Silizium und Titan können solche Materialien auch zusätzliche Elemente wie z. B. Aluminium, Zirkonium, Zinn, Eisen, Kobalt, Nickel, Gallium, Bor oder geringe Mengen an Fluor enthalten. In den mit dem erfindungsgemäßen Verfahren vorzugsweise regenerierten Zeolith-Katalysatoren kann das Titan des Zeoliths teilweise oder vollständig durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Üblicherweise stellt man die genannten Titan-, Zirkonium-, Chrom-, Niob-, Eisen- und Vanadiumzeolithe dadurch her, dass man ein Synthese-Gemisch vorlegt, d.h. eine wässrige Mischung aus einer SiO₂-Quelle, einer Titan-, Zirkonium-, Chrom-, Niob-, Eisen bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei ein zumindest teilweise kristallines Produkt entsteht. Im Rahmen der vorliegenden Erfindung wird dieser Schritt des integrierten Verfahrens zur Herstellung eines zeolithhaltigen Formkörpers als Schritt (I) bezeichnet.

Im Rahmen des Schrittes (I) wird in einer bevorzugten Ausführungsform mindestens eine Templatverbindung eingesetzt, die unter anderem bevorzugt zur Ausbildung der erwünschten Porengröße eingesetzt wird. Im Prinzip bestehen bezüglich der Templatverbindung keinerlei Beschränkungen, außer dass sie zumindest teilweise zur Porenbildung beitragen müssen. Als unter anderem geeignete Templatverbindungen sind etwa quartäre Ammoniumsalze wie beispielsweise Tetrapropylammoniumhydroxid, Tetrapropylammoniumbromid, Tetraethylammoniumhydroxid, Tetraethylammoniumbromid oder Diamine oder weitere, aus der Literatur bekannte Templatverbindungen zu nennen.

In einer weiter bevorzugten Ausführungsform wird als das mindestens eine zeolithische Material mindestens einen Zeolith, ausgewählt aus der folgenden Gruppe, erhalten: Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

### Schritt (II): Ultrafiltration

In den Verfahren nach dem Stand der Technik wird das durch die hydrothermale Umsetzung in Schritt (I) erhaltene Gemisch, welches typischerweise eine Suspension von zumindest teilweise kristallinem zeolithhaltigem Feststoff in einer Mutterlauge darstellt, anschließend nach konventionellen Methoden abfiltriert, zentrifugiert, sprühgetrocknet oder sprühgranuliert.

Im erfindungsgemäßen Verfahren findet im Anschluss an den Schritt (I) und vor dem Schritt (III) des Agglomerierens/ der Granulation ein Schritt (II) der Ultrafiltration zum Aufkonzentrieren und somit zum Erhöhen des Feststoffgehaltes statt. Im Gegensatz zum Stand der Technik erfolgt dieses Aufkonzentrieren *ohne* wesentliche(s) Granulation/Agglomerieren.

Bei den Verfahren der Ultra- und der Diafiltration handelt es sich um konvektive Verfahren, bei denen das Trennen bzw. Aufkonzentrieren von FeststoffTeilchen (Partikel) primär aufgrund der Teilchengröße erfolgt. Dabei wird ein Druckgradient entlang einer porösen Membran angelegt. Je kleiner die Porengröße der Membran, desto größer ist der zur Trennung nötige Energieaufwand, gegeben durch den anzulegenden Druckgradienten. Die Auswahl der Membran ist dabei, wie unten erläutert ist, von besonderer Bedeutung.

Im Schritt (II) wird das Gemisch aus Schritt (I), also normalerweise eine Suspension, in ein Retentat und ein Permeat aufgeteilt, wobei der Gehalt an Feststoff im Retentat höher ist als im Gemisch und der Gehalt an Feststoff im Permeat geringer ist als im Gemisch. In einer bevorzugten Ausführungsform beträgt am Ende von Schritt (II), d.h. nach mindestens einem Durchgang der Ultra- bzw. Diafiltration der Feststoff-Gehalt im Retentat 20 bis 80 Gew.-%, wobei der Feststoff-Gehalt vor Schritt (II) 1 bis 20 Gew.-% beträgt. In einer besonders bevorzugten Ausführungsform beträgt der Feststoff-Gehalt im Gemisch vor dem Schritt (II) 1 bis 20 Gew% und nach dem Schritt (II) im Retentat von 50 bis 80 Gew%. Die Angaben in Gew.-% beziehen sich dabei jeweils auf das Gesamtgewicht des Gemisches bzw. des Retentats.

Der Feststoff-Gehalt im Permeat soll dabei 5 Gew.-% nicht übersteigen, in einer bevorzugten Ausführungsform soll er 1 Gew.-% nicht übersteigen, jeweils bezogen auf das Gesamtgewicht des Permeats. In einer weiter bevorzugten Ausführungsform ist der Feststoffgehalt im Permeat so gering, dass das Permeat optisch klar ist (d.h. unter Betrachtung mit Licht der Wellenlängen von 400 nm bis 800 nm) oder dass der Feststoffgehalt durch Trocknung nicht nachgewiesen werden kann.

Um einen nennenswerten Deckschichtaufbau ("Sekundärmembran") aus dem zeolithhaltigen Feststoff auf der Membranoberfläche zu vermeiden, der zu einer deutlichen Abnahme des Permeatflusses führen würde, wird durch Umpumpen, mechanische Bewegung der Membran oder Rühraggregate zwischen den Membranen eine Relativgeschwindigkeit zwischen Membran und Suspension erzeugt, die 0,1 - 10 m/s beträgt.

Das Aufkonzentrieren kann in Batchfahrweise durch mehrmaligen Durchgang der Suspension durch die Membranmodule oder kontinuierlich durch einmaligen Durchgang durch eine oder mehrere nacheinandergeschaltete Feed- und Bleedstufen erfolgen. Weiterhin können mindestens zwei Membranen oder Membranmodule hintereinander (in Serie) oder parallel geschaltet sein.

Für das Membranverfahren werden Membrantrennschichten mit Porendurchmessern zwischen 1 nm (molekulare Trenngrenzen ca. 1 kD) und 1 µm, bevorzugt 10 nm (molekulare Trenngrenzen ca. 20 kD) bis 500 nm eingesetzt. Porendurchmesser von 50 nm bis 200 nm sind besonders bevorzugt. Die Trennschichten können aus mindestens einem Material, ausgewählt aus der folgenden Gruppe, bestehen: organische Polymere, insbesondere Cellulosederivate, Regeneratcellulose, Polyolefine, Polycarbonate, Polysulfone, Polymere mit N-C-Bindungen in der Hauptkette; Keramiken, insbesondere Silikate, Aluminiumoxide; Gläser; Metalle, insbesondere eisenhaltige Metalle und dabei insbesondere Edelstahl-Werkstoffe; Kohlenstoff-Modifikationen, insbesondere durch Pyrolyse von Kohlenstoff-Vorläuferverbindungen erhaltene Materialien, sowie Kombinationen oder Gemische aus mindestens zwei der vorstehend genannten Materialien.

Weiterhin müssen alle Materialien, aus denen die Membran besteht, im Feedmedium, d.h. im vorliegenden Fall im oben beschriebenen Synthese-Gemisch, und bei der einzustellenden Filtrationstemperatur weitestgehend inert und stabil sein. Aus mechanischen Gründen sind die Trennschichten in der Regel auf einem oder auch auf mehreren ein- oder mehrschichtigen Träger- oder Unterschichten aufgebracht, wobei diese aus dem gleichen oder auch aus unterschiedlichen Materialien, im Vergleich zur Trennschicht, bestehen können. Beispiele für mögliche Materialkombinationen sind in der folgenden Tabelle aufgeführt:

| **Trennschicht** | **Unterstruktur (gröber als Trennschicht)** |
|---|---|
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |
| Keramik: z.B. α-Al₂O₃, ZrO₂, TiO₂, SiC, gemischte keramische Werkstoffe | |
| Polymer: z.B. PP, PTFE, PVDF, Polysulfon, Polyethersulfon, | |
| Polyetheretherketon, Polyamid | |

Die Membranen können in jeder dem Fachmann bekannten Geometrie eingesetzt werden. Dabei sind Flach-, Platten-, Rohr-, Spiral-, Multikanalelement-, Kapillar-oder Wickelgeometrien bevorzugt. Es ist wesentlich, dass die gewählte Geometrie für das entsprechende Druckgehäuse, das eine Trennung zwischen Retentat (zeolithreich) und dem Permeat (zeolitharmes oder zeolithfreies Filtrat) erlaubt, geeignet ist.

Die optimalen transmembranen Drücke zwischen Retentat und Permeat sind im wesentlichen abhängig vom Durchmesser der Membranporen, den hydrodynamischen Bedingungen, die den Deckschichtaufbau beeinflussen, und der mechanischen Stabilität der Membran bei der Filtrationstemperatur. Diese Drücke betragen je nach Membranart zwischen 0,2 und 60 bar, vorzugsweise zwischen 0,5 und 20 bar. Höhere transmembrane Drücke führen in der Regel zu höheren Permeatflüssen. Dabei kann in dem Fall, in dem mehrere Module in Serie geschaltet sind, der Transmembrandruck für jeden Modul durch Anhebung des Permeatdruckes abgesenkt und damit angepasst werden.

Die Betriebstemperatur (Filtertemperatur) ist abhängig von der Membranstabilität und der Temperaturstabilität des Synthese-Gemisches. Bevorzugt liegt die Temperatur zwischen Raumtemperatur und 150°C, wobei zu beachten ist, dass das im Synthese-Gemisch befindliche Lösungsmittel nicht unvertretbar verdampft. Temperaturen zwischen 30°C und 80°C sind besonders bevorzugt.

Höhere Temperaturen führen in der Regel zu höheren Permeatflüssen. Die erreichbaren Permeatflüsse sind stark von der eingesetzten Membranart und Membrangeometrie, von den Prozessbedingungen, von der Feedzusammensetzung (im Wesentlichen der Zeolithkonzentration) abhängig. Die Permeat-Flüsse liegen typischerweise zwischen 5 und 500 kg/m²/h.

Folgende Membranen sind beispielsweise einsetzbar:

| Hersteller | Membran | Trenngrenze (kD) Porendurchmesser (nm) |
|---|---|---|
| Atech Innovations GmbH | UF / TiO₂ auf α-Al₂O₃ / 1, 2 | 20 kD |
| | UF / ZrO₂ auf α-Al₂O₃ / 1, 2 | 50 nm |
| | MF / α-Al₂O₃ auf α-Al₂O₃ / 1, 2 | 0,1; 0,2; 0,4; 0,8; 1,2 µm |
| Rhodia / Orelis | MF / ZrO₂ oder TiO₂ auf Keramik / 1, 2 | 0, 1; 0,2; 0,45; 0,8 µm |
| | UF / ZrO₂ oder TiO₂ auf Keramik / 1, 2 | 15, 50, 150; 300 kD |
| | UF / ZrO₂-TiO₂ auf Kohlenstoff / 1 | 50; 150; 300 kD |
| | MF / zrO₂-TiO₂ auf Kohlenstoff / 1 | 0,14 µm |
| Graver Technologies | UF / TiO₂auf Stahl / 1 | 100 nm |
| Bekaert | MF / Metall auf Metall | 0,2 - 1 µm |
| NADIR Filtrations GmbH | UF / Polyethersulfon oder Polysulfon/ 3 | 10 - 150 kD |
| | UF / Polyethersulfon / 1 | 40, 100 kD |
| Creavis | UF / ZrO₂ auf α-Al₂O₃ und Metall / 3 | 25, 80 nm |
| Osmonics / Desal | UF / Polysulfon / 3 | 40 nm |
| | UF/PVDF/3 | 10 kD |
| | NIF/PVDF/3 | 300 nm |
| Schumacher | UF / TiO₂ oder ZrO₂ auf Keramik / 1, 2 | 5,10 und 50 nm |
| | MF / α-Al₂O₃ auf Keramik | 100 und 200 nm |

| | | |
|---|---|---|
| 1: Rohrmembran; 2: Mehrkanalelement; 3: Flachmembran für Wickel-, Taschen-, Plattenstapel- oder Sondermodule mit bewegter Membran bzw. Rühraggregaten zwischen den Membranen; UF: Ultrafiltration; MF: Mikrofiltration | | |

### Fest-Flüssig-Trennung:

In einem weiteren, optionalen, Schritt, der sich typischerweise an den Schritt des Aufkonzentrierens durch Ultrafiltration anschließt und Teil des vorstehend definierten Schrittes (II) ist, kann der Feststoffgehalt der als Retentat erhaltenen Suspension durch konventionelle Verfahren weiter erhöht werden. Dies kann beispielsweise durch Auftrennung der erhaltenen Suspension in mehrere Teile und anschließendes Abtrennen des in einem Teil enthaltenen Feststoffes durch kuchenbildende Filtration, Zentrifugation und andere geeignete Verfahren erreicht werden.

Den so erhaltenen Filterkuchen oder das Sediment kann man anschließend, gegebenenfalls nach einem Waschschritt, in den verbliebenen Teil der Suspension zurückführen.

### Schritt (III): Agglomerieren/Granulation

Im Anschluss an den Schritt (II) des Aufkonzentrierens und/oder Abtrennens können die Feststoff-Partikel nach jedem bekannten Verfahren des Agglomerierens und/oder der Granulation vergrößert werden. Verfahrensschritte des Trocknens, die typischerweise auch zu einem zumindest teilweisen Agglomerieren/Granulieren führen und/oder sich an den Schritt des Granulierens/Agglomerierens anschließen, sind dabei explizit eingeschlossen. Solche Verfahren sind in der folgenden, nicht abschließenden, d.h. beispielhaften Aufzählung angegeben:
(i) Sprühtrocknung;
(ii) Wirbelschicht-Trocknung
(iii) Sprühtrocknung mit integrierter Wirbelschicht
(iv) batchweise Vakuum-Kontakttrocknung;
(v) Band-Trocknung
(vi) Wirbelschicht-Sprühgranulationstrocknung;
(vii) kontinuierliche Kontakttrocknung;
(viii) kontinuierliche Pastenmahltrocknung;
(ix) Mikrogranulation im Sprühturm;
(x) Agglomerieren durch Zugabe eines Bindemittels;
(xi) Agglomerieren durch Verschieben des pH-Wertes.

Bezüglich der Punkte (i) und (vi) ist der diesbezügliche Inhalt der DE- 197 31 627 bzw. der US 6 106 803 relevant Bezüglich des Punktes (xi) ist der diesbezügliche Inhalt der EP 0 638 362 B1 relevant.

Für alle Punkte (i) bis (xi) gilt, dass die Zugabe von mindestens einem Zusatzstoff vor, während oder nach bzw. vor und nach bzw. vor und während bzw. während und nach bzw. vor, während und nach dem jeweiligen Schritt der Trocknung/ Granulation/ des Agglomerierens erfolgen kann. Solche Zusatzstoff können beispielsweise aus der folgenden Gruppe entnommen sein: Bindemittel, Füllstoffe, Porenbildner. Bezüglich der Auswahl dieser Zusatzstoffe gilt das im übernächsten Abschnitt für die Formbildung Geschriebene.

In einer bevorzugten Ausführungsform werden Teile des Agglomerats/ Granulats bzw. das gesamte Agglomerat/Granulat in den Schritt (III) zurückgeführt.

### Nachbehandlung

Zur Verbesserung des katalytischen Verhaltens kann sich an den Schritt (II) oder den Schritt (III) oder an beide, jeweils optional in Verbindung mit einem Trocken- und/oder Kalzinier-Schritt, noch eine mehrmalige Waschbehandlung mit schwefelsaurer Wasserstoffperoxidlösung anschließen, worauf der Feststoff erneut getrocknet und anschließend gebrannt (kalziniert) werden kann. Daran kann sich eine Behandlung mit Alkalimetallverbindungen anschließen, um den Zeolith von der H-Form in die Kation-Form zu überführen. Der so hergestellte Feststoff kann dann, wie nachstehend beschrieben, zu einem Formkörper verarbeitet werden.

### Schritt (F): Formgebung

Das erfindungsgemäße Verfahren zur Herstellung eines zeolithhaltigen Formkörpers geht entweder vom aufkonzentrierten, optional agglomerierten Feststoff nach Schritt (II) oder (III) aus, oder von einem getrockneten und optional kalzinierten und/oder nachbehandelten agglomerierten Pulver.

In jedem Fall beinhaltet die Formgebung das Bilden einer plastischen Masse, die mindestens einen zeolithhaltigen Feststoff, sowie weiterhin ein Bindemittel, gegebenenfalls einen Porenbildner auf der Basis von in wässrigen Lösungsmitteln dispergier-, suspendier- oder emulgierbaren Polymeren, gegebenenfalls eine Mischung enthaltend mindestens einen Alkohol und Wasser, gegebenenfalls eine oder mehrere organische viskositätssteigernde Substanzen und weitere aus dem Stand der Technik bekannte Zusatzstoffe, enthält.

Die durch inniges Vermischen, insbesondere Kneten, der obigen Komponenten erhaltene plastische Masse wird vorzugsweise durch Strangpressen oder Extrudieren verformt und der erhaltene Formkörper wird nachfolgend getrocknet und abschließend kalziniert.

Als Bindemittel kann im Prinzip jede Substanz eingesetzt werden, die eine über die ohne Bindemittel ohnehin vorhandenen Physisorption hinausreichende Adhäsion und/oder Kohäsion zwischen den zu bindenden Partikeln, hier dem (pulverförmigen) Feststoff, vermittelt. Bevorzugte Bindemittel sind ausgewählt aus der folgenden Gruppe: Orthokieselsäureester, Tetraalkoxysilane, Tetraalkoxytitanate, Tetraalkoxyzirkonate oder ein Gemisch aus zwei oder mehr davon, vorzugsweise Tetramethoxysilan, Tetraethoxysilan, Tetrapropoxysilan und Tetrabutoxysilan, die analogen Tetraalkoxytitan- und -zirkonium-Verbindungen sowie Trimethoxy-, Triethoxy-, Tripropoxy-Derivate, wobei Tetramethoxysilan, Tetraethoxysilan und Kieselsole besonders bevorzugt sind. Weitere bevorzugte Bindemittel sind amphiphile Substanzen, d.h. Moleküle mit einem polaren und einem nicht-polaren Anteil, sowie Graphit.

Als bevorzugte Bindemittel zur Herstellung des erfindungsgemäßen Formkörpers werden aluminiumhaltige Bindemittel eingesetzt. Zu nennen sind hierbei insbesondere Tonmineralien, und künstlich oder natürlich hergestellte Aluminiumoxide, wie z. B. alpha-, beta-, gamma-, delta-, eta-, kappa-, chi- und theta-Aluminiumoxid und deren anorganische oder metallorganische Vorläuferverbindungen, wie z.B. Gibbsit, Bayerit, Boehmit, Pseudoboehmit, Trialkoxyaluminate, bevorzugt Aluminiumtriisopropylat.

Diese Bindemittel können entweder alleine, als Gemisch aus zwei oder mehr davon, oder zusammen mit anderen für zeolithische Materialien eingesetzten Bindemitteln, wie z.B. den oben genannten Substanzen und/oder Oxiden des Siliziums, Bors, Phosphors, Zirkoniums und/oder Titans verwendet werden. Im einzelnen sind diesbezüglich insbesondere Siliziumdioxid, wobei das SiO₂ als Kieselsol oder in Form von Tetraalkoxysilanen in den Formgebungsschritt eingebracht werden kann, sowie Tone, z.B. Montmorillonite, Kaoline, Bentonite, Halloysite, Dickite, Nacrite und Ananxite zu nennen.

Der erfindungsgemäß erhaltene Formkörper enthält vorzugsweise bis zu ungefähr 80 Gew.-%, weiter bevorzugt ungefähr 10 bis ungefähr 75 Gew.-% und insbesondere ungefähr 25 bis ungefähr 45 Gew.-% Bindemittel, jeweils bezogen auf die Gesamtmasse des Formkörpers.

Wie sich aus obigem bereits ergibt, können in jedem Fall auch Gemische aus zwei oder mehr der obengenannten Bindemittel eingesetzt werden.

Im Rahmen des erfindungsgemäßen Verfahrens können zur Einstellung einer bestimmten Porengröße, Porengrößenverteilung und Porenvolumina, falls dies gewünscht ist, noch Polymere zugegeben werden, wobei erfindungsgemäß in wässrigen Lösungsmitteln dispergier-, suspendier- oder emulgierbare Polymere für diesen Zweck eingesetzt werden.

Dabei wird das Polymer vorzugsweise ausgewählt unter polymeren Vinylverbindungen, wie z.B. Polystyrol, Polyacrylaten, Polymethacrylaten, Polyolefinen, Polyamiden und Polyestern. Diese Polymere werden beim Kalzinieren wieder weitgehend aus dem Formkörper entfernt.

Sofern vorhanden, beträgt der Gehalt an Polymer während der Herstellung des Formkörpers ungefähr 5 bis ungefähr 90 Gew.-%, vorzugsweise ungefähr 15 bis ungefähr 75 Gew.-% und insbesondere ungefähr 25 bis 55 Gew.-%, jeweils bezogen auf die Menge an zeolithhaltigem Feststoff im Gemisch.

Ferner wird bei der Herstellung des erfindungsgemäßen Formkörpers ein Anteigungsmittel eingesetzt.

Als Anteigungsmittel können alle dafür geeigneten, aus dem Stand der Technik bekannten Substanzen verwendet werden. Vorzugsweise sind dies organische, insbesondere hydrophile Polymere, wie z.B. Cellulose, Stärke, Polyacrylate, Polymethacrylate, Polyvinylalkohol, Polyvinylpyrrolidon, Polyisobuten, Polytetrahydrofuran. Diese Substanzen fördern in erster Linie die Bildung einer plastischen Masse während des Knet-, Verformungs- und Trocknungsschritts durch Verbrücken der Primärpartikel und gewährleisten darüber hinaus die mechanische Stabilität des Formkörpers beim Verformen und Trocknen. Diese Substanzen werden beim Kalzinieren wieder aus dem Formkörper entfernt.

Als weitere Zusatzstoffe können Amine oder aminartige Verbindungen, wie z.B. Tetraalkylammoniumverbindungen oder Aminoalkohole, sowie carbonathaltige Substanzen, wie z.B. Calciumcarbonat, zugesetzt werden. Derartige weitere Zusatzstoffe sind in EP-A 0 389 041, EP-A 0 200 260 und in WO 95/19222 beschrieben.

Statt basischer Zusatzstoffe ist es auch möglich, saure Zusatzstoffe zu verwenden. Diese können unter anderem eine schnellere Reaktion des Metallsäureesters (=Bindemittels) mit dem zeolithhaltigen Feststoff bewirken. Bevorzugt sind organische saure Verbindungen, die sich nach dem Verformungsschritt durch Kalzinieren herausbrennen lassen. Besonders bevorzugt sind Carbonsäuren, wie z. B. Ameisensäure. Derartige Säuren modifizieren ebenfalls die Oberflächen der hier in Rede stehenden Formkörper.

Es können weitere Zuschlagstoffe und Lösungsmittel eingesetzt werden, die zur Plastifizierung der zu formenden Masse beitragen. Derartige Lösungsmittel und Zuschlagstoffe sind dem Fachmann bekannt.

Selbstverständlich können auch Gemische aus zwei oder mehr der oben genannten Zusatzstoffe eingesetzt werden.

Die Zugabereihenfolge der Bestandteile der den Zeolithen enthaltenden Masse (Gemisch) ist nicht kritisch. Es ist sowohl möglich, zuerst das Bindemittel zuzugeben, anschließend das in Wasser dispergierbare, emulgierbare oder suspendierbare Polymer, die organische viskositätssteigernde Substanz, ggf. den Zusatzstoff und zum Schluß das Anteigungsmittel, als auch die Reihenfolge bezüglich des Bindemittels, Polymers, der organischen viskositätssteigemden Substanz und der Zusatzstoffe zu vertauschen.

Nach der Zugabe des Bindemittels zum zeoltihhaltigen Feststoff, dem gegebenenfalls die organische viskositätssteigernde Substanz bereits zugegeben worden ist, wird die in der Regel (aber nicht notwendigerweise) pulverförmige Masse 10 bis 180 Minuten im Kneter oder Extruder homogenisiert. Dabei wird in der Regel bei Temperaturen im Bereich von ungefähr 10°C bis zum Siedepunkt des Anteigungsmittel und Normaldruck oder leichtem überatmosphärischem Druck gearbeitet. Danach erfolgt die Zugabe der restlichen Bestandteile, und das so erhaltene Gemisch wird solange geknetet, bis eine verstrangbare oder extrudierfähige, plastische Masse entstanden ist.

Im Sinne der vorliegenden Erfindung sind für die Verfahren im Rahmen des Schrittes der Formgebung solche Verfahren bevorzugt, bei denen die Verformung durch Extrusion in üblichen Extrudern, beispielsweise zu Strängen mit einem Durchmesser von üblicherweise ungefähr 1 bis ungefähr 10 mm, insbesondere ungefähr 2 bis ungefähr 5 mm, erfolgt. Derartige Extrusionsvorrichtungen werden beispielsweise in Ullmann's Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Prinzipiell können jedoch zur Formgebung alle herkömmlichen Knet- und Verformungsvorrichtungen bzw. Verfahren, wie sie zahlreich aus dem Stand der Technik für die Herstellung von Formkörpern ganz allgemein bekannt sind, eingesetzt werden. Dabei können ganz allgemein die folgenden Vorgehensweisen unterschieden werden: (i) Brikettieren, d.h. mechanisches Verpressen eines pulverförmigen Materials mit oder ohne Binder und/oder anderen Materialien, (ii) Pelletieren, d.h. Kompaktieren eines feuchten oder angefeuchteten pulverförmigen Materials durch kreisförmige/rotierende Bewegungen, sowie (iii) Sintern, d.h. das zu kompaktierende Material wird einer thermischen Behandlung ausgesetzt.

Konkret kann der Formgebungs-Schritt (F) aus der folgenden Gruppe ausgewählt werden, wobei die Kombination von mindestens zwei dieser Methoden explizit eingeschlossen ist: Brikettieren durch Stempelpressen, Walzenpressen, Ringwalzenpressen, Brikettieren ohne Bindemittel; Pelletieren, Schmelzen, Spinning-Techniken, Abscheidung, Schäumen, Sprühtrocknen; Brennen im Schachtofen, Konvektionsofen, Wanderrost, Drehrohrofen, Kollern.

Das Kompaktieren kann bei Umgebungsdruck oder bei gegenüber dem Umgebungsdruck erhöhtem Druck stattfinden, beispielsweise in einem Druckbereich von 1 bar bis zu mehreren hundert bar. Weiterhin kann das Kompaktieren bei Umgebungstemperatur oder bei gegenüber der Umgebungstemperatur erhöhter Temperatur stattfinden, beispielsweise in einem Temperaturbereich von 20°C bis 300°C. Ist Trocknen und/oder Brennen Bestandteil des Formgebungsschritt, so sind Temperaturen bis zu 1500°C denkbar. Schließlich kann das Kompaktieren in der Umgebungs-Atmosphäre stattfinden oder in einer kontrollierten Atmosphäre. Kontrollierte Atmosphären sind beispielsweise Schutzgas-Atmosphären, reduzierende und/oder oxidierende Atmosphären.

### Nachbehandlung des Formkörpers und Kalzinieren:

Nach Beendigung des mindestens einen Verfahrens der Formgebung werden die erhaltenen Formkörper bei im allgemeinen ungefähr 30°C bis 140°C (1 bis 20 h, Normaldruck) getrocknet und bei ungefähr 400°C bis ungefähr 800°C (3 bis 10 h, Normaldruck) kalziniert.

Selbstverständlich können die erhaltenen Stränge bzw. Extrudate zerkleinert werden. Sie werden dabei vorzugsweise zu einem Granulat oder Splitt mit einem Partikeldurchmesser von 0,1 bis 5 mm, insbesondere 0,5 bis 2 mm zerkleinert.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Die nach dem erfindungsgemäßen Verfahren hergestellten Formkörper besitzen - verglichen mit entsprechenden Formkörpern des Standes der Technik - eine gute mechanische Stabilität bei gleichzeitiger verbesserter Aktivität und/oder Selektivität.

Neben dem oben beschriebenen Verfahren zur Herstellung eines zeolithhaltigen Feststoffes beschreibt die vorliegende Erfindung auch den besagten Feststoff an sich, und zwar erhältlich nach einem Verfahren umfassend mindestens die folgenden Schritte:
- Schritt (I):: zumindest teilweise Kristallisation eines mindestens einen Zeolithen enthaltenden Festsstoffes aus einem Synthese-Gemisch unter Erhalt des Gemisches (I), umfassend zumindest den besagten Feststoff sowie mindestens einen Hilfsstoff;
- Schritt (II):: Aufkonzentrieren des Feststoffes, der sich im Gemisch (I) befindet, durch Ultrafiltration unter Erhalt eines Retentats und eines Permeats; dieser Schritt beinhaltet optional eine Fest-Flüssig-Trennung, beispielsweise des Feststoffes von der Mutterlauge;
- Schritt (III):: Agglomerieren oder Granulation oder Agglomerieren und Granulation der Feststoff-Partikel im aufkonzentrierten Retentat aus Schritt (II)
dadurch gekennzeichnet, dass das im Schritt (II) erhaltene Permeat oder zumindest ein darin enthaltener Hilfsstoff zumindest teilweise in den Schritt (I) zurückgeführt wird; dieser Schritt beinhaltet optional das Trocknen der Feststoff-Partikel.

Optional umfasst das Verfahren weiterhin das Abtrennen und Kalzinieren oder Abtrennen oder Kalzinieren der aggregierten bzw. granulierten Feststoff-Partikel.

Weiterhin beschribt die vorliegende Erfindung einen Formkörper, der mindestens ein zeolithisches Material enthält, und der erhältlich ist aus dem oben beschriebenen Feststoff, und zwar unter Durchführung mindestens folgender Schritte;
- Schritt (F):: Formgebung anschließend an die Schritte (II) oder (III);
- Schritt (K):: Kalzinierung anschließend an die Schritte (III) oder (F).
.

Bezüglich der Teilschritte, die zur Formgebung eingesetzt werden können sowie der Bedingungen, unter denen der agglomerierte oder der nicht agglomerierte Feststoff oder der kompaktierte Feststoff kalziniert werden kann, gilt das oben Gesagte.

Schließlich beschreibt die vorliegende Erfindung noch die Verwendung des nach einem der vorstehend genannten Verfahren hergestellten zeolithhaltigen Feststoffes oder des Feststoffes oder des Formkörpers als solchen, wie gleichfalls oben beschrieben. Die erfindungsgemäßen bzw. erfindungsgemäß hergestellten, mindestens einen Zeolithen enthaltenden Feststoffe oder Formkörper können dabei insbesondere zur katalytischen Umwandlung organischer Moleküle eingesetzt werden. Umsetzungen dieser Art sind beispielsweise Oxidationen, insbesondere die Epoxidation von Verbindungen mit mindestens einer C-C-Mehrfachbindung.

Dies umfasst in einer bevorzugten Ausführungsform die Epoxidation von Olefinen, wie beispielsweise die Herstellung von Propylenoxid aus Propylen und H₂O₂, die Hydroxylierung von Aromaten, wie z.B. das Gewinnen von Hydrochinon aus Phenol und H₂O₂ oder die Umsetzung von Toluol zu Kresol, die Umwandlung von Alkanen zu Alkoholen, Aldehyden und Säuren. Weiterhin kann der in Rede stehende Katalysator verwendet werden für: Isomerisierungsreaktionen, wie z.B. die Umwandlung von Epoxiden zu Aldehyden, sowie weitere in der Literatur mit für zeolithhaltige Katalysatoren beschriebenen Umsetzungen, wie sie beispielsweise in W. Hölderich, "Zeolites: Catalysts for the Synthesis of Organic Compounds", Elsevier, Stud. Surf. Sci. Catal., 49, Amsterdam (1989), S. 69 bis 93, und insbesondere für mögliche Oxidationsreaktionen von B. Notari in Stud. Surf. Sci. Catal., 37 (1987), S. 413 bis 425, beschrieben sind.

Dabei eignen sich die vorstehend beschriebenen Feststoffe oder Formkörper, die mindestens ein zeolithisches Material enthalten, insbesondere für die Epoxidation von Olefinen mit 2 bis 8 C-Atomen, weiter bevorzugt Ethylen, Propylen oder Buten, und insbesondere Propen zu den entsprechenden Olefinoxiden. Entsprechend betrifft die vorliegende Erfindung insbesondere die Verwendung des hierin beschriebenen zeolithhaltigen Formkörpers oder Feststoffes zur Herstellung von Propylenoxid ausgehend von Propylen und Wasserstoffperoxid. Weitere Details der Reaktionsführung sind aus dem Stand der Technik wohlbekannt. Die folgenden Druckschriften der Anmelderin sind in diesem Kontext relevant: WO 01/36094, WO 01/34298, WO 01/72729, WO 01/10855, WO 00/21945.

Weiterhin beschreibt die vorliegende Erfindung die Verwendung des erfindungsgemäßen bzw. erfindungsgemäß hergestellten Formkörpers oder eines Gemisches aus zwei oder mehr davon zur Hydroxylierung von aromatischen organischen Verbindungen, zur Umwandlung von Alkanen zu Alkoholen, Ketonen, Aldehyden und Säuren, zur Ammonoximierung von Ketonen und zur Herstellung von Amin-N-Oxiden.

### Ausführungsbeispiel:

Als Membran für die Ultrafiltration nach Schritt (II) wurde eine keramische Einkanalrohrmembran der Firma Atech Innovations GmbH mit einem Außendurchmesser von 10 mm, einem Innendurchmesser von 6 mm und einer Länge von 750 mm eingesetzt. Die eigentliche filterwirksame Membran aus ZrO₂ mit einer Porenweite von 50 nm ist dabei auf der Innenseite des keramischen Rohres aus α-Al₂O₃ aufgebracht.

Die Syntheselösung enthielt ca. 6,9 % Zeolith und ca. 3,4 % Tetrapropylammoniumhydroxid (Details hierzu können der EP-B 0 991 469 entnommen werden).

Die Membran wurde in einen Pumpkreis eingesetzt, wobei der besagte Pumpkreis aus einem Vorratsgefäß, einer Pumpe, einem Wärmetauscher, einem Druckrohr für die Membran und einem Druckhalteventil besteht. Weiterhin befand sich vor der Membran eine Durchfluss-, eine Temperatur- und eine Druckmessung und nach der Membran eine Druckmessung.

Die zu konzentrierende Suspension wurde durch die Innenseite der Rohrmembran gepumpt, wobei ein Teilstrom die Membran als Permeat passierte und durch das Keramikträgermaterial abgeführt und auf einer Waage gesammelt wurde. Dabei wurden die Strömungsgeschwindigkeit der Suspension in der Rohrmembran auf 5 m/s, die Filtrationstemperatur auf 60°C und der Transmembrandruck auf 1 bar eingeregelt. Der Fluss bei der Batchkonzentrierung betrug am Start ca. 90 kg/m²/h und zum Ende bei einem Zeolithgehalt von 62,5 % und einem Tetrapropylammoniumhydroxid (TPA)-Gehalt von 3,4 % ca. 4 kg/m²/h. Der Gehalt an Feststoff (Zeolith und TPA) wurde durch Trocknung bzw. für das TPA durch Titration bestimmt. Das Permeat war jenseits der experimentellen Nachweisgrenze frei von Zeolith und enthielt Tetrapropylammoniumhydroxid in einer Konzentration von ca. 3,4 %.

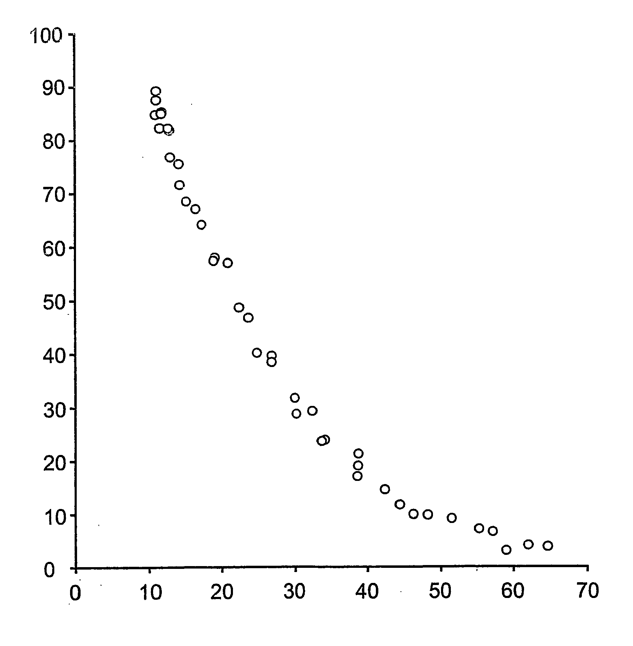

Figur 1: Der mit diesem Versuchsaufbau erzielte gesamte Feststoffgehalt (Zeolith plus TPA; angegeben in Gew.-%) ist auf der horizontalen Achse dargestellt, und zwar als Funktion des Permeatflusses (in kg/m²/h), welcher auf der vertikalen Achse aufgetragen ist.

## Patentansprüche

1. Integriertes Verfahren zur Herstellung eines mindestens einen Zeolithen enthaltenden Feststoffes, umfassend mindestens die folgenden Schritte
Schritt (I) zumindest teilweise Kristallisation eines mindestens einen Zeolithen enthaltenden Festsstoffes aus einem Synthese-Gemisch unter Erhalt des Gemisches (I), umfassend zumindest den besagten Feststoff sowie mindestens einen Hilfsstoff;
Schritt (II) Aufkonzentrieren des Feststoffes, der sich im Gemisch (I) befindet, durch Ultrafiltration unter Erhalt eines Retentats und eines Permeats;
Schritt (III) Agglomerieren oder Granulation oder Agglomerieren und Granulation der Feststoff-Partikel im Retentat aus Schritt (II),
**dadurch gekennzeichnet, dass** das im Schritt (II) erhaltene Permeat oder zumindest ein darin enthaltener Hilfsstoff zumindest teilweise in den Schritt (I) zurückgeführt wird.

2. Verfahren nach Anspruch 1, **dadurch gekennzeichnet, dass** der Feststoff-Gehalt im Gemisch vor dem Schritt (II) 1 bis 20 Gew.-% und nach dem Schritt (II) im Retentat von 50 bis 80 Gew.-% beträgt, jeweils bezogen auf das Gesamtgewicht des Gemisches bzw. des Retentats.

3. Verfahren nach Anspruch 1 oder 2, **dadurch gekennzeichnet, dass** die zur Ultrafiltration eingesetzte mindestens eine Membran Trennschichten mit Porendurchmessern von 10 nm bis 500 nm, bevorzugt von 50 bis 200 nm, enthält.

4. Verfahren nach Anspruch 3, **dadurch gekennzeichnet, dass** die Geometrie der mindestens einen Membran ausgewählt ist aus der folgenden Gruppe: Flach-, Rohr-, Multikanalelement-, Kapillar-, Wickelgeometrie.

5. Verfahren nach einem der Ansprüche 1 bis 4, **dadurch gekennzeichnet, dass** der transmembrane Druck 0,5 bis 20 bar beträgt.

6. Verfahren nach einem der Ansprüche 1 bis 5, **dadurch gekennzeichnet, dass** mindestens einer der Schritte des Agglomerierens oder der Granulation oder mindestens zwei dieser Schritte, die in Folge ausgeführt werden, aus der folgenden Gruppe ausgewählt wird/werden
(i) Sprühtrocknung
(ii) Wirbelschicht-Trocknung
(iii) Sprühtrocknung mit integrierter Wirbelschicht
(iv) batchweise Vakuum-Kontakttrocknung
(v) Band-Trocknung
(vi) Wirbelschicht-Sprühgranulationstrocknung
(vii) kontinuierliche Kontakttrocknung
(viii) kontinuierliche Pastenmahltrocknung
(ix) Mikrogranulation im Sprühturm
(x) Agglomerieren durch Zugabe eines Bindemittels
(xi) Agglomerieren durch Verschieben des pH-Wertes.

7. Verfahren nach Anspruch 6, **dadurch gekennzeichnet, dass** in den Schritten (i) bis (xi) vor, während oder nach bzw. vor und nach bzw. vor und während bzw. während und nach bzw. vor, während und nach dem jeweiligen Schritt der Trocknung/ Granulation/ des Agglomerierens mindestens ein Zusatzstoff zugegeben wird.

8. Verfahren nach einem der Ansprüche 1 bis 7, **dadurch gekennzeichnet, dass** auf den Schritt (II) oder auf den Schritt (III) ein Schritt (F) der Formgebung folgt, wobei der mindestens eine Schritt der Formgebung aus der Gruppe Brikettieren, Pelletieren, Sintern ausgewählt wird.

9. Verfahren nach Anspruch 8, wobei die Formgebung das Bilden einer plastischen Masse, die mindestens einen zeolithhaltigen Feststoff sowie weiterhin ein Bindemittel enthält, beinhaltet, wobei bevorzugte Bindemittel Tetramethoxysilan, Tetraethoxysilan und Kieselsol sind.

10. Verfahren nach einem der Ansprüche 1 bis 9, **dadurch gekennzeichnet, dass** auf mindestens einen der Schritte (II) oder (III) oder auf den Schritt (F) ein Schritt (K) des Kalzinierens bei Temperaturen größer als 400 °C erfolgt.

11. Verfahren nach einem der Ansprüche 1 bis 10, wobei der Hilfsstoff eine Templatverbindung ist.

12. Verfahren nach einem der Ansprüche 1 bis 11, wobei das Gemisch (I) eine aus einer Kristallisation erhaltene Mutterlauge, enthaltend darin suspendierten Feststoff sowie den mindestens einen Hilfsstoff, ist.

13. Verfahren nach Anspruch 12, wobei die Mutterlauge bis zu 5 Gew.-% an Partikeln größer als 2 nm enthält.

14. Verfahren nach einem der Ansprüche 1 bis 13, wobei der Zeolith ein Titanzeolith mit MFI-, MEL- oder MFI/MEL-Mischstruktur ist.

15. Verfahren nach einem der Ansprüche 1 bis 14, wobei bei der Ultrafiltration zwischen Membran und Suspension eine Relativgeschwindigkeit von 0,1 - 10 m/s durch Umpumpen, mechanische Bewegung der Membran oder Rühraggregate zwischen den Membranen erzeugt wird.

16. Verfahren nach einem der Ansprüche 1 bis 15, wobei bei der Ultrafiltration die Betriebstemperatur (Filtertemperatur) zwischen 30 °C und 80 °C liegt.

## Claims

1. An integrated process for producing a solid containing at least one zeolite, which process comprises at least the following steps:
step (I): at least partial crystallization of a solid containing at least one zeolite from a synthesis mixture to produce the mixture (I) comprising at least said solid and at least one auxiliary;
step (II): concentrating the solid present in the mixture (I) by ultrafiltration to produce a retentate and a permeate;
step (III): agglomerating or granulating or agglomerating and granulating the solid particles in the retentate from step (II), wherein the permeate obtained in step (II) or at least an auxiliary present therein is at least partially recycled into step (I).

2. The process according to claim 1, wherein the solids content in the mixture prior to step (II) is from 1 to 20% by weight, and the solids content in the retentate subsequent to step (II) is from 50 to 80% by weight, in each case based on the total weight of the mixture or the retenate, respectively.

3. The process according to claim 1 or 2, wherein the at least one membrane used for the ultrafiltration contains separating layers having pore diameters of from 10 nm to 500 nm, preferably of from 50 to 200 nm.

4. The process according to claim 3, wherein the geometry of the at least one membrane is selected from the following group: flat, tubular, multichannel-element, capillary and wound geometry.

5. The process according to one of claims 1 to 4, wherein the transmembrane pressure is from 0.5 to 20 bar.

6. The process according to one of claims 1 to 5, wherein at least one of the steps of agglomerating or granulating or at least two of these steps is/are selected from the following group:
(i) spray drying;
(ii) fluidized-bed drying;
(iii) spray drying with integrated fluidized bed;
(iv) batchwise vaccum contact drying;
(v) belt drying;
(vi) fluidized-bed spray granulation drying;
(vii) continuous contact drying;
(viii) continuous paste mill drying;
(ix) microgranulation in a spray tower;
(x) agglomeration by addition of a binder;
(xi) agglomeration by changing the pH.

7. The process according to claim 6, wherein, in steps (i) to (xi), at least one additive is added prior to, during or after, or prior to and after, or prior to and during, or during and after, or prior to, during and after the respective drying/granulating/agglomerating step.

8. The process according to one of claims 1 to 7, wherein step (II) or step (III) is followed by a shaping step (S), the at least one shaping step being selected from the group briquetting, pelletizing, sintering.

9. The process according to claim 8, wherein the shaping includes the forming of a plastic mass, comprising at least one zeolite-containing solid and also a binder, preferred binders being tetramethoxysilane, tetraethoxysilane and silica sol.

10. The process according to one of claims 1 to 9, wherein at least one of steps (II) and (III) or step (S) is followed by a calcining step (C) at temperatures in excess of 400°C.

11. The process according to one of claims 1 to 10, wherein the auxiliary is a template compound.

12. The process according to one of claims 1 to 11, wherein the mixture (I) is a mother liquor obtained by crystallization, comprising a solid suspended therein and the at least one auxiliary.

13. The process according to claim 12, wherein the mother liquor contains up to 5% by weight of particles more than 2 nm in size.

14. The process according to one of claims 1 to 13, wherein the zeolite is a titanium zeolite having the MFI, MEL or MFI/MEL mixed structure.

15. The process according to one of claims 1 to 14, wherein during ultrafiltration a relative velocity between membrane and suspension of 0.1 - 10 m/s is generated by pumped circulation, mechanical movement of the membrane or stirrers between the membranes.

16. The process according to one of claims 1 to 15, wherein during ultrafiltration the operating temperature (filter temperature) is between 30°C and 80°C.

## Revendications

1. Procédé intégré en vue de la fabrication d'une matière solide contenant au moins une zéolite, comprenant au moins les étapes suivantes
Étape (I) tout au moins cristallisation partielle d'une matière solide fait d'un mélange de synthèse, contenant au moins une zéolite, avec maintien du mélange (I), comprenant au moins la matière solide suscitée ainsi qu'au moins un adjuvant;
Étape (II) concentration de la matière solide qui se trouve, dans le mélange (I), par ultrafiltration sou maintien d'un rétentat et d'un perméat;
Étape (III) agglomération ou granulation ou agglomération et granulation des particules de matière solide dans le rétentat provenant de l'étape (II),
**caractérisé en ce que** le perméat obtenu dans l'étape (II) ou au moins un adjuvant qui y est contenu est reconduit tout au moins en partie dans l'étape (I).

2. Procédé selon la revendication 1, **caractérisé en ce que** la teneur en matière solide dans le mélange avant l'étape (II) est de 1 à 20 % en poids et **en ce qu'**elle est, après l'étape (II), dans le rétentat, de 50 à 80 % en poids, à chaque fois par rapport au poids total du mélange ou du rétentat.

3. Procédé en vue de la revendication 1 ou 2, **caractérisé en ce que** la au moins une membrane utilisée en vue de l'ultrafiltration contient des couches de séparation ayant des diamètres de pores de 10 nm à 500 nm, de préférence de 50 à 200 nm.

4. Procédé selon la revendication 3, **caractérisé en ce que** la géométrie de la au moins une membrane est sélectionnée parmi le groupe suivant: géométrie de plat, de tube, d'éléments multicanaux, de capillaires, à enroulement.

5. Procédé selon l'une quelconque des revendications 1 à 4, **caractérisé en ce que** la pression transmembranaire est de 0,5 à 20 bars.

6. Procédé selon l'une quelconque des revendications 1 à 5, **caractérisé en ce qu'**au moins une des étapes de l'agglomération ou de la granulation ou au moins deux de ces étapes, qui sont exécutées consécutivement, est ou sont sélectionnées parmi le groupe suivant
(i) Séchage par pulvérisation
(ii) Séchage à couche fluidisée
(iii) Séchage par pulvérisation avec couche fluidisée intégrée
(iv) Séchage par contact sous vide par charge
(v) Séchage à bande
(vi) Séchage de granulation à pulvérisation à couche fluidisée
(vii) Séchage par contact en mode continu
(viii) Séchage par mouture de pâte en mode continu
(ix) Micro granulation en tour de pulvérisation
(x) Agglomération par addition d'un liant
(xi) Agglomération par décalage de la valeur de pH.

7. Procédé selon la revendication 6, **caractérisé en ce que**, dans les étapes (i) à (xi) avant, pendant ou après ou avant et après ou avant et pendant ou pendant et après ou avant, pendant et après l'étape correspondante du séchage/de la granulation/de l'agglomération, l'on ajoute au moins un additif.

8. Procédé selon l'une quelconque des revendications 1 à 7, **caractérisé en ce qu'**une étape (F) de mise en forme suit l'étape (II) ou l'étape (III), la au moins une étape de mise en forme étant sélectionnée parmi le groupe mise sous forme de briquettes, mise sous forme de pastilles, frittage.

9. Procédé selon la revendication 8, la mise sous forme incluant la formation d'une masse plastique, qui contient au moins une matière solide contenant au moins une zéolite ainsi qu'en outre un liant, les liants préférés étant le tétraméthoxysilane, le tétraéthoxysilane, et un sol de silice.

10. Procédé selon l'une quelconque des revendications 1 à 9, **caractérisé en ce que** l'étape (K) de calcination à des températures supérieures à 400 °C suit au moins une des tapes (II) ou (III) ou l'étape (F).

11. Procédé selon l'une quelconque des revendications 1 à 10, l'adjuvant étant un composé modèle.

12. Procédé selon l'une quelconque des revendications 1 à 11, le mélange (I) étant une solution-mère obtenue à partir d'une cristallisation, contenant en son sein une matière solide suspendue ainsi que le au moins un adjuvant.

13. Procédé selon la revendication 12, la solution-mère contenant jusqu'à 5 % en poids de particules supérieures à 2 nm.

14. Procédé selon l'une quelconque des revendications 1 à 13, la zéolite étant une zéolite de titane ayant une structure mixte MFI, MEL ou MFI/MEL.

15. Procédé selon l'une quelconque des revendications 1 à 14, une vitesse relative de 0,1 - 10 m/s étant produite entre les membranes, au cours de l'ultrafiltration entre la membrane et la suspension, par une pompage, par un mouvement mécanique de la membrane ou grâce à des unités d'agitation.

16. Procédé selon l'une quelconque des revendications 1 à 15, la température d'exploitation (température de filtration) se situant lors de l'ultrafiltration à une valeur comprise entre 30 °C et 80 °C.
